# FASCICULE DE BREVET EUROPEEN

(11) **EP 2 854 913 B1**
(45) Date de publication et mention de la délivrance du brevet: **06.07.2016**
(21) Numéro de dépôt: 13739747.7
(22) Date de dépôt: 22.05.2013
(51) Int. Cl.: A61M 15/00

(54) **DISPOSITIF DE DISTRIBUTION DE PRODUIT FLUIDE**
VORRICHTUNG ZUR VERTEILUNG EINES FLÜSSIGEN PRODUKTS
DEVICE FOR DISTRIBUTING A FLUID PRODUCT

(30) Priorité: 24.05.2012 FR 1254784
(43) Date de publication de la demande: 08.04.2015
(73) Titulaire: Aptar France SAS, 27110 Le Neubourg (FR)
(72) Inventeur: BAILLET, Matthieu, 76240 Bonsecours (FR); COLOMB, Arnaud, 78480 Verneuil Sur Seine (FR)
(74) Mandataire: CAPRI
(86) Numéro de dépôt international: PCT/FR2013/051110
(87) Numéro de publication internationale: WO 2013/175120

(56) Documents cités:
- WO-A1-2011/154659
- WO-A2-2008/012458
- DE-A1- 10 011 717
- FR-A1- 2 881 117

## Description

La présente invention concerne un dispositif de distribution de produit fluide, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est d'abord chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Les documents FR-2 881 117, WO 2008/012458, WO 2011/154659 et DE 100 11 717 décrivent des dispositifs de l'art antérieur.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel dispositif qui soit simple et peu coûteux à fabriquer et à assembler, fiable d'utilisation, garantissant une précision et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a donc pour objet un dispositif de distribution de produit fluide, comportant un corps, au moins un réservoir individuel contenant une dose unique de produit fluide, tel que de la poudre, des moyens d'ouverture étant prévus pour ouvrir un réservoir individuel à chaque actionnement du dispositif, le dispositif comportant un embout d'inhalation et un système de déclenchement par l'inhalation qui comporte une chambre d'air déformable coopérant avec ledit embout d'inhalation et un élément déclencheur coopérant avec ladite chambre d'air, de sorte que lors d'une inhalation à travers ledit embout d'inhalation, ladite chambre d'air est déformée et ledit élément déclencheur actionne lesdits moyens d'ouverture, de sorte qu'un réservoir est ouvert par lesdits moyens d'ouverture, ladite chambre d'air comportant un corps latéral déformable, une première extrémité ouverte comportant un bord périphérique entourant une ouverture formant l'entrée de ladite poche creuse, et une seconde extrémité formant le fond de ladite poche creuse, ladite seconde extrémité comportant des moyens de connexion pour connecter ladite chambre d'air audit élément déclencheur, ledit corps latéral étant en forme de soufflet comportant plusieurs parties de soufflet, avantageusement six, disposées axialement les unes à la suite des autres, ledit corps latéral en forme de soufflet comportant des parties de soufflet ayant des diamètres extérieurs et/ou intérieurs différents.

Avantageusement, ladite chambre d'air est une poche creuse borgne comportant une seconde extrémité fermée formant le fond de ladite poche creuse.

Avantageusement, la première partie de soufflet adjacente à ladite ouverture a un diamètre extérieur inférieur au diamètre extérieur de la seconde partie de soufflet adjacente à ladite première partie de soufflet.

Avantageusement, ladite chambre d'air est connectée audit embout d'inhalation et à une chambre de distribution recevant la dose de produit contenue dans un réservoir après son ouverture, dans le chemin d'écoulement de l'inhalation, ladite chambre de distribution contenant au moins une bille mobile.

Avantageusement, le dispositif comporte des moyens de support mobiles adaptés à déplacer un réservoir individuel contre lesdits moyens d'ouverture à chaque actionnement, lesdits moyens de support mobiles étant déplaçables entre une position de non-distribution et une position de distribution, lesdits moyens de support mobiles étant sollicités vers leur position de distribution par des moyens élastiques, tel qu'un ressort ou une lame élastique, et étant retenus en position de non-distribution par des moyens de blocage qui sont libérés par l'inhalation de l'utilisateur.

Avantageusement, ledit élément déclencheur coopère avec lesdits moyens de blocage pour libérer lesdits moyens de support mobiles, qui, une fois libérés, sollicitent un réservoir contre lesdits moyens d'ouverture.

Avantageusement, lesdits moyens de support mobiles supportent une roue de guidage, les réservoirs étant réalisés sous la forme d'une bande allongée comportant plusieurs réservoirs individuels disposés les uns à la suite des autres, ladite roue de guidage faisant avancer ladite bande à chaque actionnement du dispositif.

Avantageusement, lesdits moyens d'ouverture comportent un élément de perçage adaptés à découper une paroi de fermeture du réservoir de telle sorte que la ou les partie(s) découpée(s) n'obstrue(nt) pas la ou les ouverture(s) formée(s).

Avantageusement, ladite chambre d'air est réalisée en silicone.

Avantageusement, lesdits moyens de connexion pour connecter ladite chambre d'air audit élément déclencheur comportent une projection formée sur l'extérieur de ladite chambre d'air déformable, ladite projection étant encliquetée sur ledit élément déclencheur lors de l'assemblage du dispositif.

Avantageusement, ladite projection s'encliquète dans un trou de l'élément déclencheur.

Avantageusement, ledit bord périphérique de la chambre d'air est soudé, notamment par ultrasons, sur une partie dudit corps, et/ou surmoulé sur ladite partie dudit corps.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- les figures 1 à 3 sont des vues schématiques en section transversale d'un dispositif de distribution selon un mode de réalisation avantageux de l'invention, respectivement avant ouverture, après ouverture mais avant inhalation, et après inhalation,
- les figures 4 à 6 représentent des vues schématiques partielles en section transversale, illustrant la phase d'assemblage de la chambre d'air déformable, respectivement avant mise en place de la chambre d'air sur la partie support, après cette mise en place et après fixation de la partie couvercle,
- les figures 7 à 9 sont des vues schématiques de détails de trois variantes de fixation de la chambre d'air audit corps,
- les figures 10 à 13 représentent des vues similaires à celles des figures 4 à 6, illustrant la phase d'assemblage de l'élément déclencheur sur la chambre d'air,
- la figure 14 est une vue schématique partielle en perspective similaire à la figure 13, et
- la figure 15 illustre les courbes d'effort et de déplacement en fonction de la dépression d'une chambre d'air selon un mode de réalisation avantageux de l'invention.

Sur les figures est représenté un exemple avantageux d'un inhalateur de poudre sèche. Cet inhalateur comporte un corps 10 sur lequel peuvent être montées coulissantes deux parties 11, 12 formant capot, adaptées à être ouvertes pour ouvrir et charger le dispositif. Le corps 10 peut être de forme environ arrondie comme représenté sur les figures, mais il pourrait avoir toute autre forme appropriée. Le corps 10 comporte un embout buccal ou d'inhalation 5 définissant un orifice de distribution à travers lequel l'utilisateur va inhaler lors de l'actionnement du dispositif. Cet orifice est typiquement disposé environ au centre de la partie supérieure du corps (dans la position représentée sur les dessins). Les capots 11, 12 peuvent s'ouvrir par pivotement autour d'un axe de rotation commun, ou autour de deux axes parallèles en étant engrené l'un avec l'autre. Tout autre moyen d'ouverture du dispositif est envisageable. En variante, le dispositif pourrait ne comporter qu'un seul capot au lieu de deux.

A l'intérieur du corps 10, il est prévu une bande de réservoirs individuels (non représentée dans des buts de clarté), également appelés blisters, réalisée sous forme d'une bande allongée sur laquelle les blisters sont disposés les uns derrière les autres, de manière connue. Cette bande de blisters est avantageusement constituée d'une couche ou paroi de base formant les cavités recevant les doses de poudre, et d'une couche ou paroi de fermeture qui obture de manière étanche chacun desdits blisters. Avant la première utilisation, la bande de blisters peut être enroulée à l'intérieur du corps 10, de préférence dans une partie de stockage, et des premiers moyens de déplacement de bande 40, notamment rotatifs, sont prévus pour progressivement dérouler et faire avancer cette bande de blisters.

Des seconds moyens de déplacement 50, notamment pivotants sur le corps 10, sont prévus pour amener un blister respectif dans une position de distribution à chaque actionnement du dispositif. Ces seconds moyens de déplacement sont avantageusement pivotants entre une position de non-distribution et une position de distribution, dans laquelle un blister coopère avec lesdits moyens d'ouverture.

La partie de bande comportant les blisters vides est avantageusement adaptée à s'enrouler dans un autre endroit dudit corps 10, de préférence une partie de réception, comme cela sera décrit plus en détail ci-après.

L'inhalateur comporte des moyens d'ouverture de blister 80 (seulement partiellement représentés dans des buts de clarté) comportant de préférence une aiguille de perçage et/ou de coupage de la couche de fermeture des blisters. De préférence, les moyens d'ouverture comportent un élément de perçage 80 fixe par rapport au corps 10, et contre lequel un blister respectif est déplacé à chaque actionnement par les seconds moyens de déplacement. Le blister est alors percé par ledit élément de perçage, qui pénètre dans ledit blister pour expulser la poudre au moyen du flux d'inhalation de l'utilisateur. Avantageusement, l'élément de perçage est adapté à découper une paroi de fermeture du réservoir de telle sorte que la ou les partie(s) découpée(s) n'obstrue(nt) pas la ou les ouverture(s) formée(s). Les documents WO 2006/079750 et WO 2009/007640 décrivent de tels moyens d'ouverture de blister.

Les premiers moyens de déplacement 40 sont adaptés à faire avancer la bande de blisters après chaque inhalation de l'utilisateur. Les seconds moyens de déplacement 50 sont adaptés à déplacer le blister à vider contre lesdits moyens d'ouverture lors de l'actionnement, avant chaque inhalation. Ces seconds moyens de déplacement peuvent être sollicités par un élément élastique 70, tel qu'un ressort ou tout autre élément élastique équivalent, ledit élément élastique pouvant être pré-chargé lors de l'ouverture du dispositif.

De préférence, les premiers moyens de déplacement 40 sont formés par une roue d'indexage qui reçoit et guide la bande de blisters. La suite de la description sera donc faite en référence à une telle roue d'indexage 40. Une rotation de cette roue d'indexage 40 fait avancer la bande de blisters. Avant chaque inhalation, un blister plein est toujours en position face aux moyens d'ouverture 80. Les seconds moyens de déplacement 50 peuvent comporter un organe pivotant autour d'un axe de rotation, ladite roue d'indexage 40 étant avantageusement montée rotative sur ledit organe pivotant.

Un cycle d'actionnement du dispositif peut être le suivant. Lors de l'ouverture du dispositif, les deux parties latérales 11, 12 formant capot sont écartées l'une de l'autre en pivotant sur le corps pour ouvrir le dispositif, et ainsi charger le dispositif. Dans cette position la roue d'indexage 40 ne peut pas se déplacer vers l'élément de perçage 80 car les seconds moyens de déplacement 50 sont retenus par des moyens de blocage appropriés (non représentés dans des buts de clarté). Les documents WO 2009/077700 et WO 2009/136098 décrivent de tels moyens de blocage, et sont donc intégrés dans la présente description à titre de références. Lors de l'inhalation par l'utilisateur à travers l'embout buccal, ces moyens de blocage sont débloqués, ce qui provoque alors le déplacement de ladite roue d'indexage 40 en direction de l'aiguille, et donc l'ouverture d'un blister.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture, on prévoit un système de déclenchement par l'inhalation 60 qui comporte avantageusement une chambre d'air 61 déformable sous l'effet de l'inhalation, cette chambre d'air étant adaptée à libérer les moyens de blocage. L'inhalation de l'utilisateur provoque la déformation de ladite chambre d'air déformable, libérant ainsi lesdits moyens de blocage et permettant donc le déplacement des seconds moyens de déplacement, et donc d'un blister respectif, vers sa position d'ouverture. Le blister n'est donc ouvert qu'au moment de l'inhalation, de sorte qu'il est simultanément vidé. Il n'y a donc aucun risque de perte de dose entre l'ouverture du blister et son vidage.

L'inhalateur comporte en outre une chambre de distribution ou dispersion 90 qui est destinée à recevoir la dose de poudre après ouverture d'un blister respectif. Avantageusement, cette chambre de distribution est pourvue d'au moins une bille 91, de préférence plusieurs, qui se déplace(nt) à l'intérieur de ladite chambre 90 pendant l'inhalation, notamment pour améliorer la distribution du mélange air et poudre après ouverture d'un blister, afin d'augmenter l'efficacité du dispositif.

Il peut être avantageux que les moyens d'ouverture, en particulier l'élément de perçage, soient formés directement sur ladite chambre de distribution, par exemple à l'extrémité d'un canal 95 menant à ladite chambre 90.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les blisters sont formés sur une bande allongée souple, qui, au début, est principalement stockée sous forme de bobine dans un logement de stockage à l'intérieur du corps 10 du dispositif. Avantageusement, cette bande de blisters enroulée est maintenue par des parois internes dudit logement de stockage sans que son extrémité arrière (dans le sens d'avancement de la bande de blisters) ne soit fixée par rapport audit corps 10, ce qui permet un assemblage plus aisé de cette bobine de bande de blisters à l'intérieur du dispositif.

La bande de blisters est déplacée au moyen de la roue d'indexage 40 qui présente avantageusement au moins un, de préférence plusieurs évidements, dont la forme correspond à celle des blisters. Ainsi, lorsque cette roue d'indexage 40 tourne, elle fait avancer la bande de blisters.

Bien entendu en variante ou de manière additionnelle, on pourrait utiliser d'autres moyens d'avancée de bande de blisters, par exemple en prévoyant un profil sur les bords longitudinaux latéraux de la bande de blisters, ledit profil étant adapté à coopérer avec des moyens d'entraînement appropriés. De même, des trous ménagés le long des bords latéraux de la bande de blisters pourraient également être utilisés pour faire avancer la bande de blisters au moyen de roues dentées coopérant avec lesdits trous.

Après ouverture d'un ou plusieurs blister(s), la partie de bande de blisters avec les blisters vidés doit pouvoir être stockée de manière aisée et non encombrante dans le dispositif, en évitant tout risque de blocage. Avantageusement, cette bande de blisters usée s'enroule automatiquement sur elle-même pour à nouveau former une bobine.

Selon encore un autre aspect de l'inhalateur, un dispositif de comptage ou d'indication de doses (non représenté dans des buts de clarté) est également prévu. Ce dispositif peut comporter des chiffres ou symboles inscrits directement sur la bande de blister et visibles à travers une fenêtre appropriée dans le corps 10 du dispositif. En variante, on pourrait envisager d'utiliser un compteur avec un ou plusieurs disque(s) ou anneau(x) rotatif(s) comportant des numéros ou symboles. Les documents WO 2008/012458 et WO 2011/154659 décrivent de tels compteurs. Un but de l'invention est d'éviter de compter des doses qui ne sont pas distribuées, par exemple en cas d'erreur de manipulation, ou d'une manipulation incomplète du dispositif. Il est donc souhaitable que le compteur ou indicateur ne soir actionné qu'une fois que l'utilisateur a inhalé, puisque c'est cette inhalation qui conditionne l'ouverture et la distribution de la dose de chaque blister. Avantageusement, l'actionnement du compteur se fait donc après inhalation, lorsque l'utilisateur referme le dispositif.

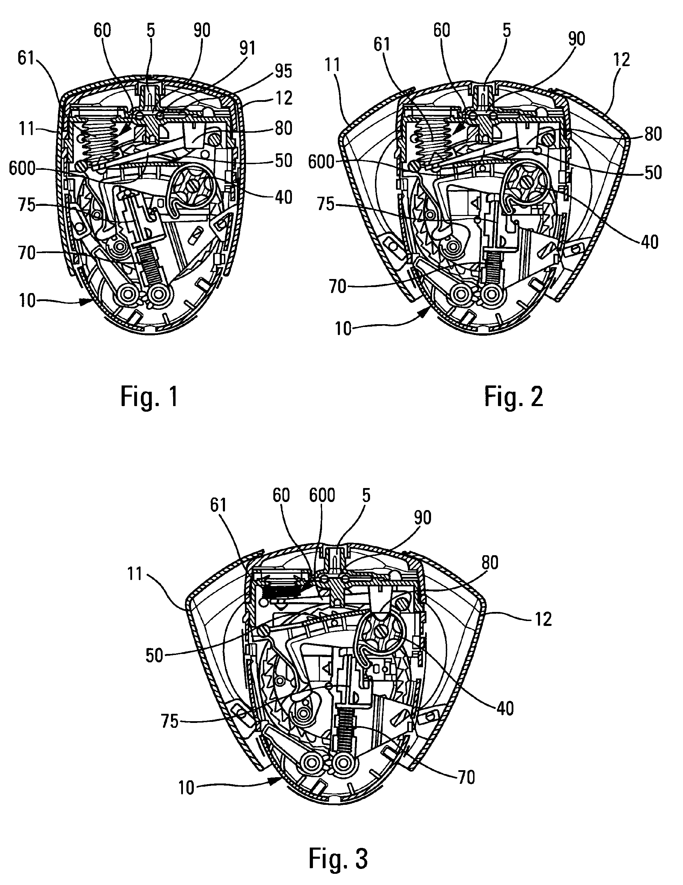

Les figures 1 à 3 représentent un cycle d'ouverture et d'inhalation du dispositif.

L'élément de capot mobile 12 est solidaire d'un organe d'armement 75 qui peut coulisser dans un logement approprié. Cet organe d'armement 75 est avantageusement pivotant par rapport audit corps 10 ensemble avec l'élément de capot 12. Cet organe d'armement 75 peut se déplacer contre le ressort 70, avantageusement un ressort à boudins, disposé dans ledit logement. L'organe d'armement 75 est donc connecté d'un côté audit ressort 70 et de l'autre côté, il coopère avec les seconds moyens de déplacement, en particulier avec un organe 50 pivotant sur le corps 10, et sur lequel est fixée de manière rotative la roue d'indexage 40.

Lorsque l'élément de capot mobile 12 est ouvert, comme représenté sur les figures 1 (position fermée) et 2 (position ouverte), l'organe d'armement 75 est déplacé dans son logement en comprimant le ressort 70. L'organe pivotant 50 des seconds moyens de déplacement est lui empêché de se déplacer par les moyens de blocage susmentionnés, qui ne seront libérés qu'au moment de l'inhalation. Ainsi, en l'absence d'inhalation dans la position ouverte de la figure 2, la fermeture des éléments de capot 11, 12 provoquerait simplement le retour à la position de repos pour l'organe d'armement 75, et la décompression du ressort 70.

Avantageusement, l'organe d'armement 75 comporte, dans sa partie en contact avec l'organe pivotant 50, une partie arrondie, telle qu'une extrémité en forme de boule, pour favoriser le glissement de l'organe d'armement sur la surface avec laquelle il coopère.

Ainsi, lorsque l'utilisateur ouvre l'inhalateur il charge le système (figures 1 & 2). S'il n'inhale pas et qu'il referme l'inhalateur, celui-ci reprend simplement sa position de départ sans déplacer la bande de blisters ni les moyens de blocage. Il n'y a donc aucun risque qu'un blister (et donc une dose de produit actif) soit perdu par un actionnement malencontreux ou incomplet dans lequel l'utilisateur n'exercerait pas d'inhalation entre l'ouverture et la fermeture.

L'ouverture du blister, son vidage, la distribution de la poudre dans les poumons de l'utilisateur, le déplacement de la bande de blisters pour amener un nouveau blister plein en face des moyens d'ouverture ainsi que le comptage de la dose ne sont donc possibles que si l'utilisateur inhale.

Les moyens de blocage, qui bloquent les seconds moyens de déplacement et notamment l'organe pivotant qui coopère avec l'organe d'armement, sont connectés à la chambre d'air déformable 61 sensible à l'inhalation de l'utilisateur, de sorte que lors de l'inhalation de l'utilisateur, ladite chambre d'air déformable se déforme, libérant lesdits moyens de blocage. Ceci permet le déplacement desdits seconds moyens de déplacement vers leur position de distribution sous l'effet de la force exercée par le ressort comprimé 70 sur l'organe d'armement 75 qui pousse sur l'organe pivotant 50. Ce déplacement provoque l'ouverture d'un blister plein et la distribution d'une dose.

Ladite chambre d'air déformable 61 du système de déclenchement par l'inhalation est une poche creuse comportant un corps latéral déformable 611, une première extrémité ouverte comportant un bord périphérique 62 entourant une ouverture formant l'entrée de ladite poche creuse, et une seconde extrémité formant le fond de ladite poche creuse.

Selon l'invention, ledit corps latéral 611 est en forme de soufflet comportant plusieurs parties de soufflet 611 a, 611b, 611 c, etc., avantageusement six, disposées axialement les unes à la suite des autres.

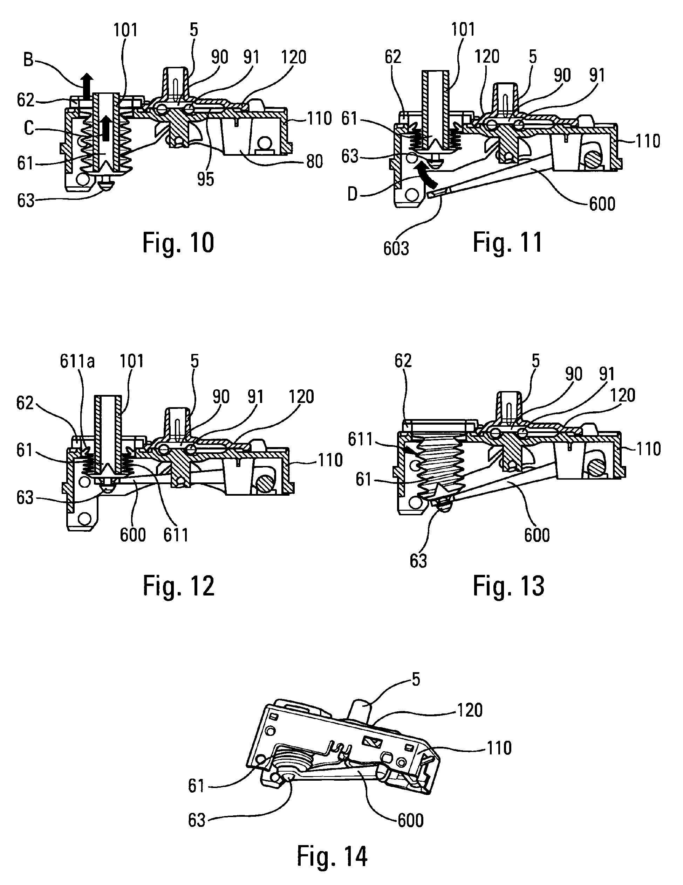

Ledit soufflet 611 comporte une ou plusieurs partie(s) de soufflet ayant des diamètres extérieurs et/ou intérieurs différents. Avantageusement, une première partie de soufflet 611 a adjacente à ladite ouverture qui a un diamètre extérieur inférieur au diamètre extérieur de la seconde partie de soufflet 611b adjacente à ladite première partie de soufflet 611 a. Ceci a notamment pour effet d'augmenter le pouvoir de compression dudit soufflet et de réduire son encombrement en position comprimée, comme visible notamment sur les figures 11 et 12, où la première partie de soufflet 611a, lorsque comprimée, est en partie déformée hors de ladite ouverture définie par ledit bord périphérique 62. Le diamètre extérieur inférieur libère ainsi la place pour la seconde partie de soufflet 611b, ce qui permet ainsi une compression supérieure dudit soufflet.

Bien entendu, une ou plusieurs autres parties de soufflet pourraient aussi avoir un diamètre extérieur différent, notamment inférieur, si nécessaire. De même, on pourrait prévoir une ou plusieurs partie(s) de soufflet avec des diamètres intérieurs différents.

La forme en soufflet fournit plusieurs avantages. Ainsi, elle définit un effort de déformation qui est axial, et donc prévisible et reproductible à chaque actionnement. Ceci permet d'obtenir une plage de déclenchement plus restreinte, avantageusement réduite d'environ 50%, dans le débit d'inhalation minimal nécessaire pour déclencher ledit système de déclenchement par l'inhalation. De plus, la forme en soufflet procure un effet ressort qui sollicite la poche d'air déformable 61 vers sa position non déformée, et favorise donc le retour du dispositif dans sa position de repos.

Avantageusement, la chambre d'air déformable 61 est une poche creuse borgne avec ladite seconde extrémité de la chambre d'air 61 qui est fermée. Ceci améliore l'étanchéité du système de déclenchement par l'inhalation, en évitant les fuites dans le fond de la chambre d'air.

Un élément déclencheur 600 coopère avec ladite chambre d'air 61, de telle sorte que lors d'une inhalation à travers ledit embout d'inhalation 5, ladite chambre d'air 61 est déformée, déplaçant ledit élément déclencheur 600 qui actionne alors lesdits moyens d'ouverture 80, notamment en libérant les moyens de blocage. Ainsi, lors d'une inhalation à travers l'embout 5, un réservoir est ouvert par lesdits moyens d'ouverture.

Ladite seconde extrémité fermée de la chambre d'air 61 comporte des moyens de connexion 63 pour connecter ladite chambre d'air audit élément déclencheur 600.

Avantageusement, ledit élément déclencheur est une tige connectée d'une part à ladite chambre d'air 61 et d'autre part aux moyens de blocage. Ainsi, lorsque la chambre d'air se déforme, elle déplace, notamment par pivotement, ladite tige, ce qui va provoquer la libération des moyens de blocage.

Lesdits moyens de connexion 63 pour connecter ladite chambre d'air 61 audit élément déclencheur 600 comportent de préférence une projection 63 formée sur l'extérieur de ladite chambre d'air déformable 61, ladite projection étant encliquetée sur ledit élément déclencheur lors de l'assemblage du dispositif, notamment dans une ouverture 603 dudit élément déclencheur.

Avantageusement, ledit bord périphérique 62 de la chambre d'air 61 est soudé, notamment par ultrasons, sur une partie 120 dudit corps 10, et/ou surmoulé sur ladite partie 120 dudit corps 10.

La chambre d'air 61 est de préférence réalisée en silicone, mais d'autres matériaux appropriés sont envisageables. L'épaisseur de la membrane est avantageusement comprise entre 0,1 mm et 0,3 mm, de préférence environ 0,2 mm.

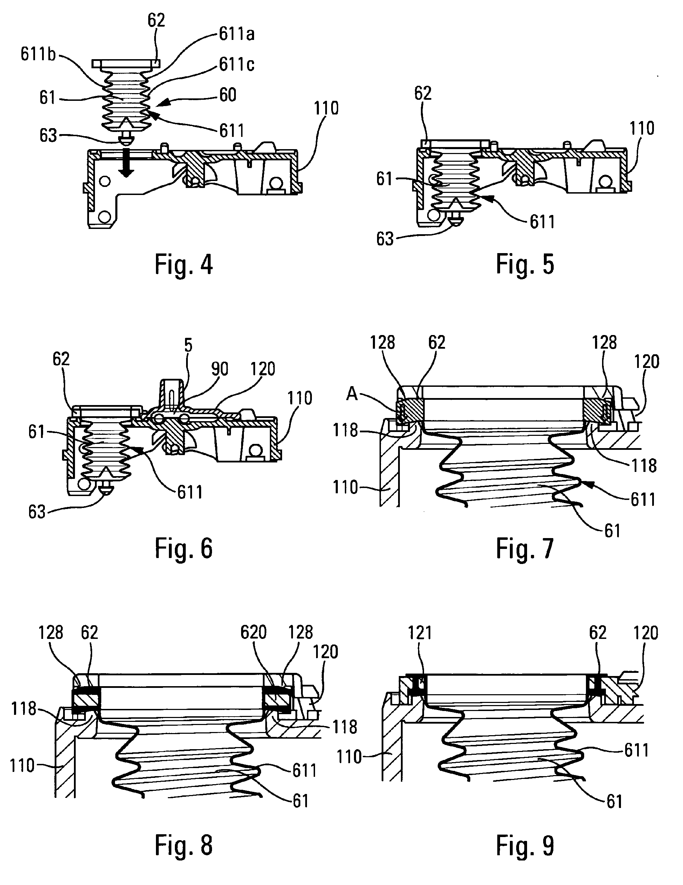

Les figures 4 à 6 illustrent un assemblage avantageux de la chambre d'air 61 dans le corps. Dans cette variante, la chambre d'air 61 est disposée dans une ouverture de réception prévue à cet effet dans une première partie de corps 110, le bord périphérique 62 de la chambre d'air 61 venant reposer sur le bord 118 de ladite ouverture de réception comme visible sur la figure 5. Une seconde partie de corps 120 est ensuite assemblée, notamment soudée par ultrasons, sur la première partie de corps 110, comme représenté sur la figure 6. Le bord périphérique 62 de la chambre d'air est alors coincé entre les deux parties de corps 110, 120.

Avantageusement, pour renforcer l'étanchéité lors de cet assemblage, la seconde partie de corps 120 peut comporter une projection ou profil axial 128 décalée radialement vers l'extérieur par rapport au bord 118 sur lequel repose le bord périphérique 62 de la chambre d'air. Cette projection 128 est adaptée à déformer ledit bord périphérique 62 de la chambre d'air 61 lors de l'assemblage, comme schématisé par la flèche A dans la figure 7.

Eventuellement, comme visible sur la figure 8, un insert rigide 620 peut être disposé dans ledit bord périphérique 62 pour renforcer sa rigidité et donc faciliter son assemblage et améliorer son étanchéité.

En variante à l'assemblage des figures 4 à 6, le bord périphérique 62 de la chambre d'air 61 peut aussi être fixé, notamment par surmoulage, directement sur la seconde partie de corps 120, comme visible sur la figure 9. Dans ce cas, la chambre d'air 61 est assemblée dans l'ouverture de la première partie de corps 110 au moment où la seconde partie de corps 120 est fixée sur ladite première partie de corps 110.

Les figures 10 à 14 illustrent l'assemblage de l'élément déclencheur 600 sur la chambre d'air 61. Après assemblage de ladite chambre d'air 61 dans le corps, un manchon d'assemblage 101 est introduit dans ladite chambre d'air 61, et ladite chambre d'air est déformée dans sa position comprimée, notamment par aspiration, comme schématisé par les flèches B et C de la figure 10. L'élément déclencheur 600, avantageusement sous forme d'une tige pivotante, est alors déplacé selon la flèche D sur la figure 11, pour encliqueter un trou 603 de ladite tige sur la projection 63 de la chambre d'air 61, comme représenté sur la figure 12. L'aspiration dans la chambre d'air 61 est alors supprimée et le manchon d'assemblage 101 retiré, ce qui provoque la déformation de la chambre d'air 61 vers son état non comprimé et le pivotement en retour de la tige 600, comme visible sur les figures 13 et 14.

Comme visible sur la figure 13, le pivotement de la tige 600 provoque une déformation de la chambre d'air 61 qui n'est pas exactement axiale.

L'utilisation d'une forme de soufflet optimise la capacité de compression de la chambre d'air et présente l'avantage de garantir une reproductibilité améliorée de la déformation par rapport à la dépression crée lors de l'inhalation, ce qui rend le déclenchement du dispositif plus fiable.

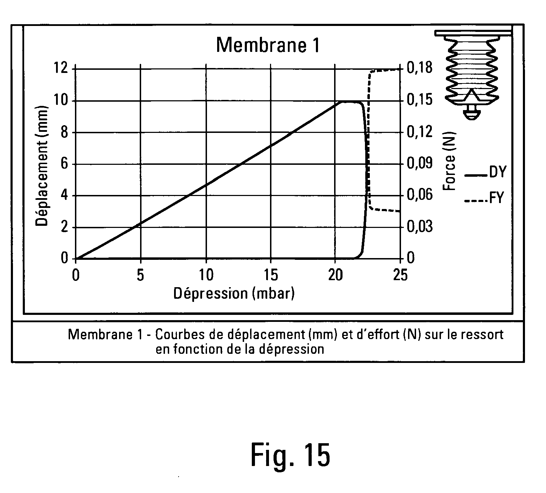

En particulier, le fait d'avoir une chambre fermée et une forme de soufflet donne des courbes d'effort et de déplacement linéaires par rapport à la dépression, contrairement à des chambres d'air de formes différentes, par exemple formée par une membrane lisse déformable, où ces courbes sont beaucoup plus aléatoires. La figure 15 illustre la linéarité de ces courbes pour une chambre d'air selon l'invention.

Dans tous les modes de réalisation décrits ci-dessus, la bande de blisters est formée par une bande présentant deux extrémités.

En variante, on pourrait utiliser une bande continue. D'autres modifications sont également possibles sans sortir du cadre de la présente invention.

La présente invention permet donc de fournir un inhalateur de poudre sèche qui procure notamment les fonctions suivantes :
▪ une pluralité de doses individuelles de poudre stockées dans des blisters individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération pré-chargé ;
▪ des moyens d'entraînement de forme appropriée en prises avec les blisters pour réaliser le déplacement de la bande de blisters après chaque inhalation, et amener un nouveau blister plein dans une position dans laquelle il est destiné à être ouvert par les moyens d'ouverture appropriés ;
▪ des moyens pour éviter une perte de dose en cas d'ouverture de l'inhalateur mais en l'absence d'inhalation ;
▪ un indicateur de doses adapté à compter les doses seulement en cas d'inhalation.

D'autres fonctions sont également fournies par le dispositif de l'invention tel que cela a été décrit précédemment.

Il est à noter que les différentes fonctions, même si elles ont été représentées comme prévues simultanément sur l'inhalateur, pourraient être mises en oeuvre séparément les unes des autres.

En particulier, le mécanisme de déclenchement par inhalation pourrait être utilisé indépendamment du type de moyens d'ouverture de réservoir, indépendamment de l'utilisation d'un indicateur de doses, indépendamment de la manière dont les blisters individuels sont arrangés les uns par rapport aux autres, etc.

Les moyens d'armement et le système de déclenchement par l'inhalation pourraient être réalisés différemment.

Il en est de même des autres parties constitutives du dispositif.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Dispositif de distribution de produit fluide, comportant un corps (10), au moins un réservoir individuel contenant une dose unique de produit fluide, tel que de la poudre, des moyens d'ouverture (80) étant prévus pour ouvrir un réservoir individuel à chaque actionnement du dispositif, le dispositif comportant un embout d'inhalation (5) et un système de déclenchement par l'inhalation (60) qui comporte une chambre d'air déformable (61) coopérant avec ledit embout d'inhalation (5) et un élément déclencheur (600) coopérant avec ladite chambre d'air (61), de sorte que lors d'une inhalation à travers ledit embout d'inhalation (5), ladite chambre d'air (61) est déformée et ledit élément déclencheur (600) actionne lesdits moyens d'ouverture (80), de sorte qu'un réservoir est ouvert par lesdits moyens d'ouverture, ladite chambre d'air (61) comportant un corps latéral déformable (611), une première extrémité ouverte comportant un bord périphérique (62) entourant une ouverture formant l'entrée de ladite poche creuse, et une seconde extrémité formant le fond de ladite poche creuse, ladite seconde extrémité comportant des moyens de connexion (63) pour connecter ladite chambre d'air audit élément déclencheur (600), ledit corps latéral (611) étant en forme de soufflet comportant plusieurs parties de soufflet (611a, 611b, 611c, ...), avantageusement six, disposées axialement les unes à la suite des autres, **caractérisé en ce que** ledit corps latéral (611) en forme de soufflet comporte des parties de soufflet ayant des diamètres extérieurs et/ou intérieurs différents.

2. Dispositif selon la revendication 1, dans lequel ladite chambre d'air (61) est une poche creuse borgne comportant une seconde extrémité fermée formant le fond de ladite poche creuse.

3. Dispositif selon la revendication 1 ou 2, dans lequel la première partie de soufflet (611 a) adjacente à ladite ouverture a un diamètre extérieur inférieur au diamètre extérieur de la seconde partie de soufflet (611 b) adjacente à ladite première partie de soufflet (611 a).

4. Dispositif selon l'une quelconque des revendications précédentes, dans lequel ladite chambre d'air (61) est connectée audit embout d'inhalation (5) et à une chambre de distribution (90) recevant la dose de produit contenue dans un réservoir après son ouverture, dans le chemin d'écoulement de l'inhalation, ladite chambre de distribution (90) contenant au moins une bille mobile (91).

5. Dispositif selon l'une quelconque des revendications précédentes, comportant des moyens de support mobiles (50) adaptés à déplacer un réservoir individuel contre lesdits moyens d'ouverture (80) à chaque actionnement, lesdits moyens de support mobiles (50) étant déplaçables entre une position de non-distribution et une position de distribution, lesdits moyens de support mobiles (50) étant sollicités vers leur position de distribution par des moyens élastiques (70), tel qu'un ressort ou une lame élastique, et étant retenus en position de non-distribution par des moyens de blocage qui sont libérés par l'inhalation de l'utilisateur.

6. Dispositif selon la revendication 5, dans lequel ledit élément déclencheur (600) coopère avec lesdits moyens de blocage pour libérer lesdits moyens de support mobiles (50), qui, une fois libérés, sollicitent un réservoir contre lesdits moyens d'ouverture (80).

7. Dispositif selon l'une quelconque des revendications précédentes, dans lequel lesdits moyens de support mobiles (50) supportent une roue de guidage (40), les réservoirs étant réalisés sous la forme d'une bande allongée comportant plusieurs réservoirs individuels disposés les uns à la suite des autres, ladite roue de guidage (40) faisant avancer ladite bande à chaque actionnement du dispositif.

8. Dispositif selon l'une quelconque des revendications précédentes, dans lequel lesdits moyens d'ouverture (80) comportent un élément de perçage adaptés à découper une paroi de fermeture du réservoir de telle sorte que la ou les partie(s) découpée(s) n'obstrue(nt) pas la ou les ouverture(s) formée(s).

9. Dispositif selon l'une quelconque des revendications précédentes, dans lequel ladite chambre d'air (61) est réalisée en silicone.

10. Dispositif selon l'une quelconque des revendications précédentes, dans lequel lesdits moyens de connexion (63) pour connecter ladite chambre d'air (61) audit élément déclencheur (600) comportent une projection (63) formée sur l'extérieur de ladite chambre d'air déformable (61), ladite projection étant encliquetée sur ledit élément déclencheur lors de l'assemblage du dispositif.

11. Dispositif selon la revendication 10, dans lequel ladite projection (63) s'encliquète dans un trou (603) de l'élément déclencheur (600).

12. Dispositif selon l'une quelconque des revendications précédentes, dans lequel ledit bord périphérique (62) de la chambre d'air (61) est soudé, notamment par ultrasons, sur une partie (120) dudit corps (10), et/ou surmoulé sur ladite partie (120) dudit corps (10).

## Patentansprüche

1. Ausgabevorrichtung für ein fluides Produkt, aufweisend einen Körper (10), mindestens einen einzelnen Behälter, der eine einzige Dosis eines fluiden Produktes, wie ein Pulver, enthält, Öffnungsmittel (80), die zum Öffnen eines einzelnen Behälters bei jeder Betätigung der Vorrichtung vorgesehen sind, wobei die Vorrichtung einen Inhalationsansatz (5) und ein durch Inhalation ausgelöstes System (60) aufweist, welches eine mit dem Inhalationsansatz (5) zusammenwirkende verformbare Luftkammer (61) und ein mit der Luftkammer (61) zusammenwirkendes Auslöseelement (600) aufweist, so dass die Luftkammer (61) bei einer Inhalation durch den Inhalationsansatz (5) verformt wird und das Auslöseelement (600) die Öffnungsmittel (80) derart betätigt, dass ein Behälter durch die Öffnungsmittel geöffnet wird, wobei die Luftkammer (61) einen seitlichen verformbaren Körper (611) aufweist, wobei ein offenes erstes Ende einen Umfangsrand (62) aufweist, der eine Öffnung umgibt, die den Eingang der hohlen Tasche umgibt, und ein zweites Ende den Boden der hohlen Tasche bildet, wobei das zweite Ende Verbindungsmittel (63) zum Verbinden der Luftkammer mit dem Auslöseelement (600) aufweist, wobei der seitliche Körper (611) in Form eines Blasebalgs vorliegt, der mehrere, vorteilhafterweise sechs, Blasebalgteile (611a, 611b, 611c, ...) aufweist, die axial aufeinander folgend angeordnet sind, **dadurch gekennzeichnet, dass** der seitliche Körper (611) in Form eines Blasebalgs Blasebalgteile aufweist, welche unterschiedliche Außendurchmesser und/oder Innendurchmesser aufweisen.

2. Vorrichtung nach Anspruch 1, wobei die Luftkammer (61) eine hohle Blindtasche ist, die ein geschlossenes zweites Ende aufweist, welches den Boden der hohlen Tasche bildet.

3. Vorrichtung nach Anspruch 1 oder 2, wobei der erste, an die Öffnung angrenzende Blasebalgteil (611 a) einen Außendurchmesser aufweist, der kleiner ist als der Außendurchmesser des zweiten Blasebalgteils (611 b), der an den ersten Blasebalgteil (611a) angrenzt.

4. Vorrichtung nach einem der vorhergehenden Ansprüche, wobei die Luftkammer (61) mit dem Inhalationsansatz (5) und mit einer Ausgabekammer (90) verbunden ist, die die in einem Behälter enthaltene Produktdosis nach dessen Öffnen in dem Inhalationsfließweg aufnimmt, wobei die Ausgabekammer (90) mindestens eine bewegliche Kugel (91) enthält.

5. Vorrichtung nach einem der vorhergehenden Ansprüche, aufweisend bewegliche Trägermittel (50), die dazu geeignet sind, bei jeder Betätigung einen einzelnen Behälter gegen die Öffnungsmittel (80) zu verschieben, wobei die beweglichen Trägermittel (50) zwischen einer Nicht-Ausgabeposition und einer Ausgabeposition verschiebbar sind, wobei die beweglichen Trägermittel (50) durch elastische Mittel (70), wie eine Feder oder ein elastisches Blatt, gegen ihre Ausgabeposition vorgespannt sind und durch Sperrmittel in ihrer Nicht-Ausgabeposition gehalten werden, die durch Inhalation des Benutzers freigegeben werden.

6. Vorrichtung nach Anspruch 5, wobei das Auslöseelement (600) mit den Sperrmitteln zusammenwirkt, um die beweglichen Trägermittel (50) freizugeben, die, sobald sie freigegeben sind, einen Behälter gegen die Öffnungsmittel (80) vorspannen.

7. Vorrichtung nach einem der vorhergehenden Ansprüche, wobei die beweglichen Trägermittel (50) ein Führungsrad (40) tragen, wobei die Behälter in Form eines länglichen Streifens ausgeführt sind, der mehrere einzelne Behälter umfasst, die aufeinander folgend angeordnet sind, wobei das Führungsrad (40) bei jeder Betätigung der Vorrichtung den Streifen vorwärtsbewegt.

8. Vorrichtung nach einem der vorhergehenden Ansprüche, wobei die Öffnungsmittel (80) ein Durchstoßelement aufweisen, das dazu geeignet ist, eine Verschlusswand des Behälters derart abzuschneiden, dass der abgeschnittene Teil (die abgeschnittenen Teile) nicht die gebildete Öffnung(en) behindert (behindern).

9. Vorrichtung nach einem der vorhergehenden Ansprüche, wobei die Luftkammer (61) aus Silikon gefertigt ist.

10. Vorrichtung nach einem der vorhergehenden Ansprüche, wobei die Verbindungsmittel (63) zum Verbinden der Luftkammer (61) mit dem Auslöseelement (600) einen Vorsprung (63) aufweisen, der an der Außenseite der verformbaren Luftkammer (61) gebildet ist, wobei der Vorsprung bei der Montage der Vorrichtung auf dem Auslöseelement eingerastet wird.

11. Vorrichtung nach Anspruch 10, wobei der Vorsprung (63) in ein Loch (603) des Auslöseelements (600) einrastet.

12. Vorrichtung nach einem der vorhergehenden Ansprüche, wobei der Umfangsrand (62) der Luftkammer (61) insbesondere durch Ultraschall auf einen Teil (120) des Körpers (10) geschweißt ist und/oder auf den Teil (120) des Körpers (10) aufgegossen ist.

## Claims

1. A fluid dispenser device including a body (10) and at least one individual reservoir containing a single dose of fluid, such as powder, opening means (80) being provided for opening an individual reservoir each time the device is actuated, the device including an inhalation piece (5) and an inhalation trigger system (60) that comprises a deformable air chamber (61) that co-operates with said inhalation piece (5), and a trigger element (600) that co-operates with said air chamber (61), such that during inhalation through said inhalation piece (5), said air chamber (61) is deformed and said trigger element (600) actuates said opening means (80), so that a reservoir is opened by said opening means, said air chamber (61) comprising: a deformable side body (611); an open first end including a peripheral edge (62) that surrounds an opening forming the inlet of said hollow pouch; and a second end forming the bottom of said hollow pouch; said second end including connection means (63) for connecting said air chamber to said trigger element (600), said side body (611) being in the form of a bellows comprising a plurality of bellows portions (611a, 611b, 611c, ...), advantageously six, arranged axially one after another, the fluid dispenser device being **characterized in that** said bellows-forming side body (611) comprises bellows portions having outside and/or inside diameters that are different.

2. A device according to claim 1, wherein said air chamber (61) is a blind hollow pouch including a closed second end that forms the bottom of said hollow pouch.

3. A device according to claim 1 or claim 2, wherein the first bellows portion (611a) adjacent to said opening has an outside diameter that is smaller than the outside diameter of the second bellows portion (611b) adjacent to said first bellows portion (611a).

4. A device according to any preceding claim, wherein said air chamber (61) is connected to said inhalation piece (5) and to a dispenser chamber (90) which receives the dose of fluid contained in a reservoir after it has been opened into the inhalation flow path, said dispenser chamber (90) containing at least one movable ball (91).

5. A device according to any preceding claim, including movable support means (50) that are adapted to move an individual reservoir against said opening means (80) on each actuation, said movable support means (50) being displaceable between a non-dispensing position and a dispensing position, said movable support means (50) being urged towards their dispensing position by resilient means (70), such as a spring or a spring blade, and being held in their non-dispensing position by blocking means that are released by the user inhaling.

6. A device according to claim 5, wherein said trigger element (600) co-operates with said blocking means so as to release said movable support means (50), which, once released, urge a reservoir against said opening means (80).

7. A device according to any preceding claim, wherein said movable support means (50) support a guide wheel (40), the reservoirs being made in the form of an elongate strip comprising a plurality of individual reservoirs disposed one behind another, said guide wheel (40) causing said strip to advance each time the device is actuated.

8. A device according to any preceding claim, wherein said opening means (80) include a perforator element that is adapted to cut a closure wall of the reservoir in such a manner that the cut portion(s) does/do not obstruct the opening(s) that is/are formed.

9. A device according to any preceding claim, wherein said air chamber (61) is made of silicone rubber.

10. A device according to any preceding claim, wherein said connection means (63) for connecting said air chamber (61) to said trigger element (600) comprise a projection (63) that is formed on the outside of said deformable air chamber (61), said projection being snap-fastened on said trigger element while the device is being assembled.

11. A device according to claim 10, wherein said projection (63) is snap-fastened in a hole (603) of the trigger element (600).

12. A device according to any preceding claim, wherein said peripheral edge (62) of the air chamber (61) is heat-sealed, in particular by ultrasound, on a portion (120) of said body (10) and/or overmolded on said portion (120) of said body (10).
